(19) Europäisches Patentamt
European Patent Office
Office européen des brevets

(11) Veröffentlichungsnummer: **0 047 448**
**B1**

# (12) EUROPÄISCHE PATENTSCHRIFT

(45) Veröffentlichungstag der Patentschrift: **21.09.83**

(51) Int. Cl.³: **C 10 M 3/14**, C 07 D 319/16, C 07 D 317/72

(21) Anmeldenummer: **81106721.4**

(22) Anmeldetag: **28.08.81**

## (54) Kraftübertragungsfluide.

(30) Priorität: **06.09.80 DE 3033658**
**28.07.81 DE 3129621**

(43) Veröffentlichungstag der Anmeldung: **17.03.82 Patentblatt 82/11**

(45) Bekanntmachung des Hinweises auf die Patenterteilung: **21.09.83 Patentblatt 83/38**

(84) Benannte Vertragsstaaten: **DE FR GB IT NL SE**

(56) Entgegenhaltungen:
**FR-A-845 122**
**US-A-2 305 228**

(73) Patentinhaber: **BAYER AG, Zentralbereich Patente, Marken und Lizenzen, D-5090 Leverkusen 1, Bayerwerk (DE)**

(72) Erfinder: **Hentschel, Karl-Heinz, Dr., Heckschenstrasse 89, D-4150 Krefeld 10 (DE)**
Erfinder: **Dhein, Rolf, Dr., Deswatinesstrasse 30, D-4150 Krefeld 1 (DE)**
Erfinder: **Winter, Hans, Prof. Dr., Eugen-Kalkschmidt-Weg 7, D-8000 München 81 (DE)**
Erfinder: **Vojacek, Herbert, Dipl. Ing., Budapester Strasse 11, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Kraftübertragungsfluide

Die Erfindung betrifft Kraftübertragungsfluide, die cyclische Ketale enthalten, die sich von alicyclischen Ketonen ableiten.

In geschmierten Traktionsgetrieben benötigt man spezielle Fluide, mit deren Hilfe das Drehmoment des Antriebsteils auf den Abtriebsteil übertragen wird. Fluide für geschmierte Traktionsgetriebe und ihre Prüftests sind bekannt und werden beispielsweise in der DE-B 1 644 926, DE-A 1 925 826, DE-A 2 506 735, SR-A 2 171 988, US-A 3 394 603, US-A 3 595 796, US-A 3 597 358 und US-A 3 997 617 beschrieben.

Um Reibkräfte zwischen dem Antriebsteil zu übertragen, ist ein Flüssigkeitsfilm aus einem Schmiermittel in der Berührungszone zwischen den beiden Wälzkörpern erforderlich, der zur Übertragung der Reibkräfte einer Scherbewegung unterworfen wird.

Eine wesentliche Rolle bei der Beurteilung von Fluiden auf ihre Eignung als Kraftübertragungsfluide spielen die Reibungszahl (auch Traktionskoeffizient genannt) und die Grösse des Schlupfes, der auftritt, wenn bei der Reibkraftübertragung die Umfangsgeschwindigkeiten des Antriebs- und des Abtriebskörpers verschieden sind [Konstruktion 31, 2 bis 6 und 55 bis 62 (1979)].

Die Reibungszahl wird bei der Anwendung in Traktionsgetrieben im wesentlichen durch den Schlupf, durch die Umfangsgeschwindigkeiten des Antriebskörpers und der Anpresskraft zwischen dem Antriebs- und Abtriebskörper bestimmt.

Die Reibzahl ist definiert als Quotient aus der bei der Reibung entstehenden Reibkraft und der Anpresskraft zwischen dem Antriebs- und Abtriebskörper. Der Schlupf ist definiert als der absolute Wert des Quotienten aus der Differenz der beiden Umfangsgeschwindigkeiten der Wälzkörper und der grösseren Umfangsgeschwindigkeit.

Bei Traktionsgetrieben wird angestrebt, das in dem Bereich eines kleinem Schlupfes (darunter versteht man einen Schlupf von kleiner als 5%) die Reibungszahl mit zunehmendem Schlupf rasch ansteigt. Hierdurch erzielt man einen guten Wirkungsgrad des Traktionsgetriebes.

Ausserdem wird angestrebt, dass die maximale Reibungszahl möglichst gross ist, um eine maximale Ausnutzung der Kraftübertragung zu erreichen.

Als Kraftübertragungsfluide werden in der DE-B 1 644 926 organische, kondensierte, gesättigte Verbindungen genannt, die aus 2 bis 9 kondensierten Ringen mit insgesamt 9 bis 60 Kohlenstoffatomen bestehen, wobei bis zu 8 dieser Atome durch Sauerstoff-, Stickstoff-, Phosphor- und/oder Siliciumatome ersetzt sein können. Als besonders geeignete Kraftübertragungsfluide werden in der DE-B 1 644 926 Decalin, 1,2'-Hydrindan, Perhydrophenanthren, Perhydrofluoren, Perhydrofluorenthen, Perhydroacenaphthen, Cyclohexyldecalin, primäres Perhydrocyclopentadien und Methylenhydrophenanthren genannt.

Es wurden neue Kraftübertragungsfluide gefunden, die spirocyclische Ketale der Formel (I)

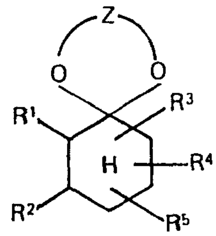

(I)

in der

$R^1$ in dem Fall, dass $R^2$ Wasserstoff bedeutet, für einen gegebenenfalls durch Niederalkyl, Niederalkoxy und/oder Cycloalkyl mit 5 bis 7 Kohlenstoffatomen substituierten Cyclohexylrest steht, oder in der

$R^1$ und $R^2$ über 4 Methylengruppen, die gegebenenfalls durch Niederalkyl, Niederalkoxy und/oder Cycloalkyl mit 5 bis 7 Kohlenstoffatomen substituiert sind, verbunden sind und einen weiteren alicyclischen Ring bilden;

$R^3$, $R^4$ und $R^5$ gleich oder verschieden sind und Wasserstoff, Niederalkyl, Niederalkoxy oder Cycloalkyl mit 5 bis 7 Kohlenstoffatomen bedeuten, und

Z gegebenenfalls durch Niederalkyl und/oder Cycloalkyl mit 5 bis 7 Kohlenstoffatomen substituiertes Alkylen mit 2 bis 4 Kohlenstoffatomen bedeutet, enthalten.

Die Grundkörper der spirocyclischen Ketale, die sich von Cyclohexyl-cyclohexanon und/oder von 1-Decalonen ableiten, können durch Niederalkyl, Niederalkoxy und/oder Cycloalkyl mit 4 bis 7 Kohlenstoffatomen substituiert sein.

Erfindungsgemäss bedeutet Niederalkyl einen geradkettigen oder verzweigten Kohlenwasserstoffrest mit mit 1 bis etwa 6 Kohlenstoffatomen. Beispielsweise seien genannt Methyl, Ethyl, Propyl, Isopropyl, Butyl, Isobutyl, Pentyl, Isopentyl, Hexyl und Isohexyl.

Erfindungsgemäss bedeutet Niederalkoxy aliphatischen Etherrest, dessen aliphatischer Teil aus einem geradkettigen oder verzweigten Kohlenwasserstoffrest mit 1 bis etwa 6 Kohlenstoffatomen besteht. Beispielsweise seien die folgenden Alkoxyreste genannt: Methoxy, Ethoxy, Propoxy, Isopropoxy, Butoxy, Isobutoxy, Pentoxy, Isopentoxy, Hexoxy und Isohexoxy. Cycloalkyl mit 5 bis 7 Kohlenstoffatomen steht für Cyclopentyl, Cyclohexyl und Cycloheptyl, bevorzugt für Cyclohexyl.

Als erfindungsgemässe Kraftübertragungsfluide werden spirocyclische Ketale der Formel (II)

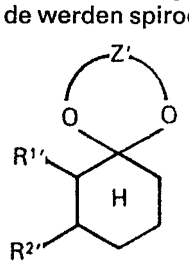

(II)

in der
$R^{1'}$ in dem Fall, dass $R^{2'}$ Wasserstoff bedeutet, für einen Cyclohexylrest steht, oder in der
$R^{1'}$ und $R^{2'}$ über 4 Methylengruppen verbunden sind und einen weiteren alicyclischen Ring bilden und
Z ' für die Gruppen

$-CH_2-CH_2-$,
$-CH_2-CH_2-CH_2-$,

$CH_3$
|
$-CH_2-C-CH_2-$ oder
|
$CH_3$

$CH_3$
|
$-CH_2-CH_2-CH-$

steht, bevorzugt.
Insbesondere werden erfindungsgemäss Kraftübertragungsfluide bevorzugt, die 7-Cyclohexyl-1,4-dioxa-spiro(5.5)-undecan, 6-Cyclohexyl-1,4-dioxa-spiro(4.5)-decan, 7-Cyclohexyl-3,3-dimethyl-1,5-dioxa-spiro(5.5)-undecan oder Spiro-[1,3-dioxan-(2.1')-decalin] enthalten.

Es ist selbstverständlich möglich, dass die erfindungsgemässen Kraftübertragungsfluide auch Gemische der genannten spirocyclischen Ketale enthalten.

Die erfindungsgemäss zu verwendenden spirocyclischen Ketale können durch Ketalisierung von substituiertem oder vorzugsweise unsubstituiertem Decalon bzw. Cyclohexyl-cyclohexanon mit 1,2-, 1,3- oder 1,4-Alkandiolen nach an sich bekannten Synthesemethoden hergestellt werden [Houben-Weyl VI/3, 204–270 (1966) und Houben-Weyl VII/1, 413–488 (1954)].

Unsubstituierte oder substituierte 1-Decalone sind leicht aus den entsprechenden 1-Naphtholen durch vollständige Hydrierung mit nachfolgender Oxidation der sekundären Alkohole zu den Ketonen erhältlich. Die entsprechenden $\alpha$-Naphthole können durch Niederalkylrste ($C_1$ bis etwa $C_6$) oder durch $C_5$-$C_7$-Cycloalkylreste substituiert sein. Beispielsweise seien die folgenden 1-Decalone genannt: 1-Naphthol, Methyl-1-naphthole wie 2-Methyl-1-naphthol, 4-methyl-1-naphthol, Dimethyl-1-naphthole wie 2,4-Dimethyl-1-naphthol, 5,8-Dimethyl-1-naphthol, Isobutyl-1-naphthole wie 2-Isobutyl-1-naphthol, 4-Isobutyl-1-naphthol, Cyclopentyl-1-naphthole wie 2-Cyclopentyl-1-naphthol, 4-Cyclopentyl-1-naphthol und Cyclohexyl-1-naphthole wie 2-Cyclohexyl-1-naphthol, 4-Cyclohexyl-1-naphthol.

Bevorzugt ist das aus 1-Naphthol leicht zugängliche unsubstituierte 1-Decalon.

Als 2-Cyclohexyl-cyclohexanone können alle zum gesättigten Keton hydrierten Aldolkondensationsprodukte zweier gegebenenfalls mit Niederalkyl ($C_1$ bis etwa $C_6$), Niederalkoxy ($C_1$ bis etwa $C_6$) oder Cyclohexyl mit 5 bis 7 Kohlenstoffatomen substituierte Cyclohexanone verwendet werden, sofern mindestens eines der Ausgangs-Cyclohexanone mindestens eine der Carboxylgruppe benachbarte freie Methylengruppe aufweist.

Cyclohexanone, die als Ausgangsverbindungen für die erfindungsgemässen spirocyclischen Ketale verwendet werden können, sind an sich bekannt (Beilstein System-Nr. 612, Band 7, Hauptwerk S. 8–32, I 6–27, II 5–36, III 14–134).

Sie müssen durch eine Aldolkondensationsreaktion mit nachgeschalteter partieller Hydrierung zunächst in substituierte 2-Cyclohexylcyclohexanone überführt werden.

Beispielsweise seien als Ausgangscyclohexanone für die Aldolkondensation die folgenden Cyclohexanone genannt: Methylcyclohexanone wie 2-Methylcyclohexanone, 3-Methylcyclohexanone, 4-Methylcyclohexanone, Dimethylcyclohexanone wie 3,4-Dimethylcyclohexanone, 3,5-Dimethylcyclohexanone, 4,4-Dimethylcyclohexanone (mit Ausnahme des 2,6-Dimethylcyclohexanons) und 3,3,5-Trimethylcyclohexanon.

Besonders bevorzugt wird als zu ketalisierendes Keton das unsubstituierte 2-Cyclohexyl-cyclohexanon, das aus dem als Nebenprodukt der technischen Adipinsäureherstellung durch Oxidation von Cyclohexanol/Cyclohexanon-Gemischen anfallende 2-Cyclohexenyl-2-Cyclohexyliden-Cyclohexanon-Gemisch besonders zugänglich ist.

Alkandiole für die Herstellung der spirocyclischen Ketale sind an sich bekannt [Houben-Weyl VI 13, 213–220 (1966)].

Beispielsweise seien die folgenden Alkandiole genannt: Ethylenglykol, Propandiol-1,3, Propandiol-1,2, Butandiol-1,2, -1,3, -1,4, Pentandiol-1,2, -1,3, -1,4, -2,3 oder -2,4, 2,2-Dimethyl-propandiol-1,3, 2-Methyl-2-Ethyl-propandiol-1,3, 2-Methyl-2-propyl-propandiol-1,3, 2-Methyl-2-butyl-propandiol-1,3, und $C_6$-$C_{12}$-Alkandiole, in welchen die beiden Hydroxylgruppen durch 2 bis 4 dazwischenliegende Kohlenstoffatome voneinander getrennt sind.

Nach einem bevorzugten Ketalisierungsverfahren setzt man das Keton mit einer äquimolaren Mischung eines Niederalkyl-orthocarbonsäureesters, z.B. Trimethylorthoformiat, und dem Alkandiol um, wobei nach Erhitzen unter Säurekatalyse und Abspaltung von Niederalkyl-monoalkoholen und Niederalkyl-carbonsäureester das gewünschte spirocyclische Ketal in besonders guter Ausbeute entsteht.

Die Herstellung der spirocyclischen Ketale erfolgt im allgemeinen im Temperaturbereich von 20 bis 250°C, bevorzugt von 50 bis 100°C.

Es kann vorteilhaft sein, die Ketalisierung in Gegenwart von katalytischen Mengen einer Säure, z.B. Schwefelsäure, durchzuführen.

Die spirocyclischen Ketale eignen sich erfindungsgemäss hervorragend als Kraftübertragungsfluide für Traktionsgetriebe. Für die Anwendung der erfindungsgemässen Kraftübertragungsfluide seien beispielsweise die folgenden Traktionsgetriebe genannt:

Umschlingungsgetriebe wie Schubgliederkettengetriebe und Rollenkettengetriebe, und Wälzgetriebe wie Kugel-Scheiben-Getriebe, Wälz-Toroidgetriebe und Kugel-Rollen-Getriebe.

Die in Traktionsgetrieben mit den erfindungsgemässen spirocyclischen Ketalen als Kraftübertragungsfluide maximal ausnutzbaren Reibungszahlen liegen im allgemeinen im Bereich von 0,05 bis 0,1, bevorzugt von 0,065 bis 0,09. Hierdurch ist eine optimale Kraftübertragung in den Getrieben gewährleistet. Im Vergleich zu handelsüblichen Kraftübertragungsfluiden wird mit Hilfe der erfindungsgemässen Fluide eine Minimierung der Getriebedimensionen ermöglicht.

Ausserdem zeichnen sich die erfindungsgemässen Fluide im Bereich eines Schlupfes von 0 bis 1% durch einen besonders starken Anstieg der Reibungszahlen aus. Damit können die bei der Kraftübertragung auftretenden Verluste sehr klein gehalten werden. Dies hat zur Folge, dass die bei der Kraftübertragung entstehende Reibungswärme gering bleibt.

Die erfindungsgemässen spirocyclischen Ketale haben vorteilhafterweise eine hohe Wärmebeständigkeit und verändern sich auch bei starker Belastung nicht.

Die Viskositätsindices der erfindungsgemässen spirocyclischen Ketale liegt im Bereich von −500 bis +100, bevorzugt von −200 bis +60. Da nur geringe Reibungswärme auftritt, ändert sich die Viskosität des Fluids bei der Anwendung im Getriebe praktisch nicht.

Die erfindungsgemässen spirocyclischen Ketale zeigen auch gute Schmierstoffeigenschaften. Daher ist bei ihrer Verwendung als Kraftübertragungsfluide gleichzeitig für eine ausreichende Schmierung des Getriebes gesorgt.

Die erfindungsgemässen Kraftübertragungsfluide können ausser den spirocyclischen Ketalen je nach Anwendungsgebiet noch weitere Komponenten enthalten. Im allgemeinen enthalten die erfindungsgemässen Kraftübertragungsfluide mindestens 50 Gew.-% eines spirocyclischen Ketals. Es ist aber auch möglich, dass sie aus dem praktisch reinen spirocyclischen Ketal ohne zusätzliche Additive bestehen. Bevorzugt enthalten die erfindungsgemässen Kraftübertragungsfluide 60 bis 95 Gew.-% des spirocyclischen Ketals.

Die möglichen Zusätze zu den spirocyclischen Ketalen in den erfindungsgemässen Kraftübertragungsfluiden hängen im wesentlichen von den Anwendungsgebieten ab. Bevorzugte Anwendungsgebiete der erfindungsgemässen Kraftübertragungsfluide sind Traktionsgetriebe.

Bei der Verwendung in Traktionsgetrieben sind die erfindungsgemässen spirocyclischen Ketale im allgemeinen die Hauptkomponente. Übliche Zusätze sind beispielsweise Viskositätsverbesserer wie Poly(meth)acrylat, Polyisobutene, hydrierte Styrol-Dien-Blockpolymeriste und hydrierte Styrol-Olefin-Copolymerisate.

Extreme Pressure und/oder Antiverschleissadditive wie Zinkdialkyl-dithiophosphat,

Antioxidantien wie Alkylphenole, Diarylamine, Phenylendiamine, Phenothiazine und organische Phosphor(III)-Verbindungen und

Farbstoffe wie Azofarbstoffe und Triphenylmethanfarbstoffe.

Die Kraftübertragungsfluide für Traktionsgetriebe bestehen im allgemeinen aus 60 bis 95 Gew.-Teilen des spirocyclischen Ketals, mit 0,1 bis 15 Gew.-Teilen des Viskositätsverbesserers, 0,1 bis 5 Gew.-Teilen des Extreme-Pressure und/oder Antiverschleissadditivs und 0,01 bis 3 Gew.-Teilen des Antioxidans. Bevorzugte Kraftübertragungsfluide für Traktionsgetriebe bestehen aus 80 bis 90 Gew.-Teilen des spirocyclischen Ketals, 0,5 bis 10 Gew.-Teilen des Viskositätsverbesserers, 0,5 bis 3 Gew.-Teilen des Extreme-Pressure und/oder Antiverschleissadditivs und 0,05 bis 2 Gew.-Teilen des Antioxidans.

Ausserdem ist es möglich, dass die Fluide 0,01 bis 1, bevorzugt 0,05 bis 0,5, Gew.-Teile eines Farbstoffs enthalten.

Im Vergleich zu bekannten handelsüblichen Kraftübertragungsfluiden zeigen die erfindungsgemässen spirocyclische Ketale enthaltenden Kraftübertragungsfluide wesentlich verbesserte Eigenschaften. Diese Verbesserung der Eigenschaften war aufgrund des Standes der Technik nicht vorauszusehen.

Beispiel 1

7-Cyclohexyl-1,5-dioxa-spiro(5.5)-undecan

1260 g (7 Mol) 2-Cyclohexyl-cyclohexanon werden in einem 4 l-Dreihalskolben mit Rührer, Destillationskolonne und Destillationsaufsatz mit Rückflussregelung zusammen mit 816,2 g Trimethylorthoformiat (7,7 Mol), 585,2 g entwässertem 1,3-Propandiol (7,7 Mol) und 7 Tropfen conc. $H_2SO_4$ unter leichtem Stickstoffstrom am Rückfluss gekocht. Dabei wird langsam ein Gemisch von Methanol und Methylformiat überdestilliert (insgesamt ca. 870 g). Danach wird abgekühlt, 3,5 g wasserfreies Kaliumcarbonat zugegeben und 10 Minuten aufgekocht. Nach dem Abfiltrieren wird zunächst im Wasserstrahlvakuum, dann im Ölpumpenvakuum fraktioniert.

Ausbeute: 968 g (58% der Theorie);
$Kp_1 = 121°C$, $n_D^{20}$: 1,4946.

Die Elementaranalyse und das NMR-Spektrum stehen im Einklang mit der Struktur und der Zusammensetzung $C_{15}H_{26}O_2$.

Kinematische Viskositäten:
bei 37,8°C (100°F) 22,6 mm²/s
bei 98,9°C (210°F) 3,39 mm²/s

Viskositätsindex: −53

Messungen der Reibzahlen (Traktionskoeffizienten) bei 50°C an einem Zweischeibenprüfstand bei unterschiedlichen Anpresskräften und Scheibenumfangsgeschwindigkeiten ergeben folgende Werte:

bei 125 N Anpresskraft:
0,987 (12,6 m/s) - 0,098 (0,84 m/s)
bei 700 N Anpresskraft:

0,098 (12,6 m/s) - 0,113 (0,84 m/s)
bei 2000 N Anpresskraft:
0,097 (12,6 m/s) - 0,107 (0,84 m/s)
bei 4000 N Anpresskraft:
0,107 (12,6 m/s) - 0,113 (0,84 m/s)

Dieses Beispiel belegt die hohen Reibungszahlen, die die erfindungsgemässen Fluide aufweisen.

Beispiel 2
6-Cyclohexyl-1,4-dioxa-spiro(4.5)-decan
Analog dem Beispiel 1 werden 1260 g (7 Mol) 2-Cyclohexyl-cyclohexanon mit 816,2 g Trimethylorthoformiat (7,7 Mol) und 477,4 g wasserfreiem 1,2-Ethandiol (7,7 Mol) umgesetzt.

Ausbeute: 1013 g (65% der Theorie);
$Kp_{0,4}$ = 99°C, $n_D^{20}$ = 1,4902

Die Elementaranalyse und das NMR-Spektrum stehen im Einklang mit der Struktur und der Zusammensetzung $C_{14}H_{24}O_2$.

Kinematische Viskositäten:
bei 37,8°C (100°F) 9,82 mm²/s
bei 98,9°C (210°F) 2,32 mm²/s
Viskositätsindex: 37

Messung der Reibzahlen (wie bei Beispiel 1):

bei 2000 N Anpresskraft:
0,085 (12,6 m/s) - 0,096 (0,84 m/s)

Beispiel 3
7-Cyclohexyl-3,3-dimethyl-1,5-dioxa-spiro(5.5)-undecan
Analog dem Beispiel 1 werden 1170 g 2-Cyclohexyl-cyclohexanon (6,5 Mol) mit 757,9 g Trimethylorthoformiat (7,15 Mol) und 743,6 g wasserfreiem 2,2-Dimethyl-propandiol-1,3 (7,15 Mol) umgesetzt:

Ausbeute: 1230 g (71% der Theorie);
$Kp_{0,3}$ = 122°C, $n_D^{20}$ = 1,4880

Die Elementaranalyse und das NMR-Spektrum stehen im Einklang mit der Struktur und der Zusammensetzung $C_{17}H_{30}O_2$.

Kinematische Viskositäten:
bei 37,8°C (100°F) 55,2 mm²/s
bei 98,9°C (210°F) 3,94 mm²/s
Viskositätsindex: −474

Messung der Reibzahlen (wie bei Beispiel 1):

bei 2000 N Anpresskraft:
0,099 (12,6 m/s) - 0,108 (0,84 m/s)
bei 4000 N Anpresskraft:
0,098 (12,6 m/s) - 0,102 (0,84 m/s).

Beispiel 4
Spiro-1,3-dioxan-(2.1')-decalin
Analog dem Beispiel 1 werden 1444 g 1-Decalon (9,5 Mol) mit 1107,7 g Trimethylorthoformiat (10,45 Mol) und 794,2 g 1,3-Propandiol (10,45 Mol) umgesetzt.

Ausbeute: 1292 g (65% der Theorie);
$Kp_{1,3}$ = 103°C, $n_D^{20}$ = 1,4944.

Die Elementaranalyse und das NMR-Spektrum stehen im Einklang mit der Struktur und der Zusammensetzung $C_{13}H_{22}O_2$.

Kinematische Viskositäten:
bei 37,8°C (100°F) 8,77 mm²/s
bei 98,9°C (210°F) 2,24 mm²/s
Viskositätsindex: 60

Messung der Reibzahlen (wie bei Beispiel 1):

bei 125 N Anpresskraft:
0,073 (12,6 m/s) - 0,092 (0,84 m/s)
bei 700 N Anpresskraft:
0,087 (12,6 m/s) - 0,106 (0,84 m/s)
bei 2000 N Anpresskraft:
0,083 (12,6 m/s) - 0,100 (0,84 m/s)
bei 4000 N Anpresskraft:
0,087 (12,6 m/s) - 0,102 (0,84 m/s)

Beispiel 5
7-Cyclohexyl-2-methyl-1,5-dioxan-spiro(5.5)-undecan
1260 g (7 Mol) 2-Cyclohexyl-cyclohexanon werden in einem 4 l-Dreihalskolben mit Rührer, Destillationskolonne und Destillationsaufsatz mit Rückflussregelung zusammen mit 816,2 g Trimethylorthoformiat (7,7 Mol), 693,0 g getrocknetem 1,3-Butandiol (7,7 Mol) und 7 Tropfen konzentrierter Schwefelsäure unter leichtem Stickstoffstrom am Rückfluss gekocht. Dabei wird langsam ein Gemisch von Methanol und Methylformiat überdestilliert (insgesamt etwa 870 g). Danach wird abgekühlt, 3,5 g wasserfreies Kaliumcarbonat zugegeben und 10 Minuten wieder erwärmt. Nach dem Abfiltrieren wird zunächst im Wasserstrahlvakuum, dann im Ölvakuum fraktioniert destilliert.

Ausbeute: 1077 g (61% der Theorie);
Kp = 105°C, $n_D^{20}$ = 1,4852

Die Elementaranalyse und das NMR-Spektrum stehen im Einklang mit der Struktur und der Zusammensetzung.

Kinematische Viskositäten:
bei 37,8°C 20,6 mm²/s
bei 98,9°C 2,91 mm²/s
Viskositätsindex: −183

Messung der Reibzahlen (wie bei Beispiel 1):

bei 125 N Anpresskraft:
0,077 (12,6 m/s) - 0,093 (0,84 m/s)
bei 400 N Anpresskraft:
0,092 (12,6 m/s) - 0,10 (0,84 m/s)
bei 1000 N Anpresskraft:

0,095 (12,6 m/s) - 0,103 (0,84 m/s)
bei 2000 N Anpresskraft:
0,097 (12,6 m/s) - 0,103 (0,84 m/s)
bei 4000 N Anpresskraft:
0,095 (12,6 m/s) - 0,100 (0,84 m/s)

Beispiel 6

In einem Zwei-Scheiben-Reibungsprüfstand nach K. Stössel [Konstruktion 31, (1979) Seite 4 und 5] wird die Abhängigkeit der Reibungszahl $\mu$ vom Schlupf s bestimmt.

Die Scheiben bestehen aus einem Werkstoff der nach DIN 17006 mit 100 Cr 6 beschrieben wird. Die Scheiben haben einen Durchmesser von 80 mm. Der arithmetrische Mittenrankwert Ra (center Line average CLA) der Antriebsscheibe beträgt 0,060 µm, die der Abtriebsscheibe 0,065 µm.

Das Fluid nach Beispiel 1 wird bei einer Einspritztemperatur von 50°C und einer Viskosität von 10,0 Pa.s zwischen die beiden Scheiben gespritzt.

In den Figuren 1 bis 4 wird die Abhängigkeit der Reibungszahlen vom Schlupf und in Abhängigkeit von der Anpresskraft (auch Last genannt) und Umfangsgeschwindigkeit der Antriebsscheibe bestimmt.

Die in den folgenden Figuren dargestellten Ergebnisse wurden bei den folgenden Anpresskräften durchgeführt:

Figur 1 $F_N$ = 125 N
Figur 2 $F_N$ = 700 N
Figur 3 $F_N$ = 2000 N
Figur 4 $F_N$ = 4080 N

Ausserdem wurde in den einzelnen Prüfungen die Umfangsgeschwindigkeit des Antriebs variiert. Die folgenden Bezeichnungen entsprechen den folgenden Umfangsgeschwindigkeiten:

1 v = 0,42 m/s
2 v = 0,84 m/s
3 v = 2,10 m/s
4 v = 4,19 m/s
5 v = 8,38 m/s
6 v = 12,57 m/s

Die Ergebnisse zeigen, dass im Bereich eines kleinen Schlupfes die Reibungszahl sehr stark zunimmt und ausserdem hohe Werte annimmt. Hierdurch erreicht man einen guten Wirkungsgrad eines Traktionsgetriebes.

**Patentansprüche**

1) Kraftübertragungsfluide, enthaltend spirocyclische Ketale der Formel (I)

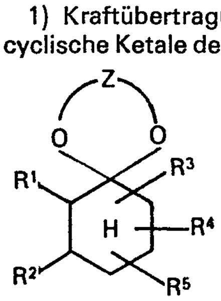
(I)

in der

$R^1$ in dem Fall, dass $R^2$ Wasserstoff bedeutet, für einen gegebenenfalls durch Niederalkyl, Niederalkoxy und/oder Cycloalkyl mit 5 bis 7 Kohlenstoffatomen substituierten Cyclohexylrest steht, oder in der

$R^1$ und $R^2$ über 4 Methylengruppen, die gegebenenfalls durch Niederalkyl, Niederalkoxy und/oder Cycloalkyl mit 5 bis 7 Kohlenstoffatomen substituiert sind, verbunden sind und einen weiteren alicyclischen Ring bilden;

$R^3$, $R^4$ und $R^5$ gleich oder verschieden sind und Wasserstoff, Niederalkyl, Niederalkoxy oder Cycloalkyl mit 5 bis 7 Kohlenstoffatomen bedeuten, und

Z gegebenenfalls durch Niederalkyl und/oder Cycloalkyl mit 5 bis 7 Kohlenstoffatomen substituiertes Alkylen mit 2 bis 4 Kohlenstoffatomen bedeutet.

2) Kraftübertragungsfluide nach Anspruch 1, enthaltend mindestens 50 Gew.-% des spirocyclischen Ketals.

3) Kraftübertragungsfluide nach den Ansprüchen 1 und 2, enthaltend 60 bis 95 Gew.-% des spirocyclischen Ketals.

4) Verwendung der Kraftübertragungsfluide nach Anspruch 1 in Getrieben.

5) Verwendung der Kraftübertragungsfluide nach Anspruch 1 in Traktionsgetrieben.

6) Verwendung nach den Ansprüchen 4 und 5 in Umschlingungsgetrieben.

7) Verwendung nach den Ansprüchen 4 und 5 in Wälzgetrieben.

**Revendications**

1. Fluides de transmission de force, contenant des cétals spirocycliques de formule (I)

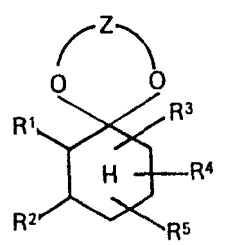
(I)

dans laquelle

$R^1$ représente, au cas où $R^2$ est l'hydrogène, un reste cyclohexyle éventuellement substitué par un radical alkyle inférieur, alkoxy inférieur et/ou cycloalkyle ayant 5 à 7 atomes de carbone, ou bien

$R^1$ et $R^2$ sont liés par quatre groupes méthylène qui sont substitués éventuellement par des radicaux alkyle inférieurs, alkoxy inférieurs et/ou cycloalkyle ayant 5 à 7 atomes de carbone, et forment un autre noyau alicyclique;

$R^3$, $R^4$ et $R^5$ sont égaux ou différents et représentent l'hydrogène, un radical alkyle inférieur, alkoxy inférieur ou cycloalkyle ayant 5 à 7 atomes de carbone, et

Z désigne un groupe alkylène ayant 2 à 4 atomes de carbone, éventuellement substitué par un radical alkyle inférieur et/ou cycloalkyle ayant 5 à 7 atomes de carbone.

2. Fluides de transmission de force suivant la revendication 1, contenant au moins 50% en poids du cétal spirocyclique.

3. Fluides de transmission de force suivant les revendications 1 et 2, contenant 60 à 95% en poids du cétal spirocyclique.

4. Utilisation des fluides de transmission de force selon la revendication 1 dans des mécanismes.

5. Utilisation des fluides de transmission de force suivant la revendication 1 dans des mécanismes de traction.

6. Utilisation suivant les revendications 4 et 5 dans des mécanismes d'enroulement.

7. Utilisation suivant les revendications 4 et 5 dans des mécanismes de roulement.

**Claims**

1. Power transmission fluids containing spirocyclic ketals of the formula (I)

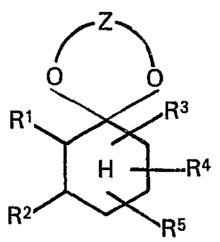

(I)

in which

$R^1$ in the case where $R^2$ denotes hydrogen, represents a cyclohexyl radical optionally substituted by lower alkyl, lower alkoxy and/or cycloalkyl with 5 to 7 carbon atoms, or in which

$R^1$ and $R^2$ are linked through 4 methylene groups, which are optionally substituted by lower alkyl, lower alkoxy and/or cycloalkyl with 5 to 7 carbon atoms and form a further alicyclic ring;

$R^3$, $R^4$ and $R^5$ are identical or different and denote hydrogen, lower alkyl, lower alkoxy or cycloalkyl with 5 to 7 carbon atoms, and

Z denotes alkylene with 2 to 4 carbon atoms which is optionally substituted by lower alkyl and/or cycloalkyl with 5 to 7 carbon atoms.

2. Power transmission fluids according to Claim 1, containing at least 50% by weight of the spirocyclic ketal.

3. Power transmission fluids according to Claims 1 and 2, containing 60 to 95% by weight of the spirocyclic ketal.

4. Use of the power transmission fluid according to Claim 1 in drive mechanisms.

5. Use of the power transmission fluids according to Claim 1 in traction drive mechanisms.

6. Use according to Claims 4 and 5 in flexible drive mechanisms.

7. Use according to Claims 4 and 5 in rolling contact drive mechanisms.

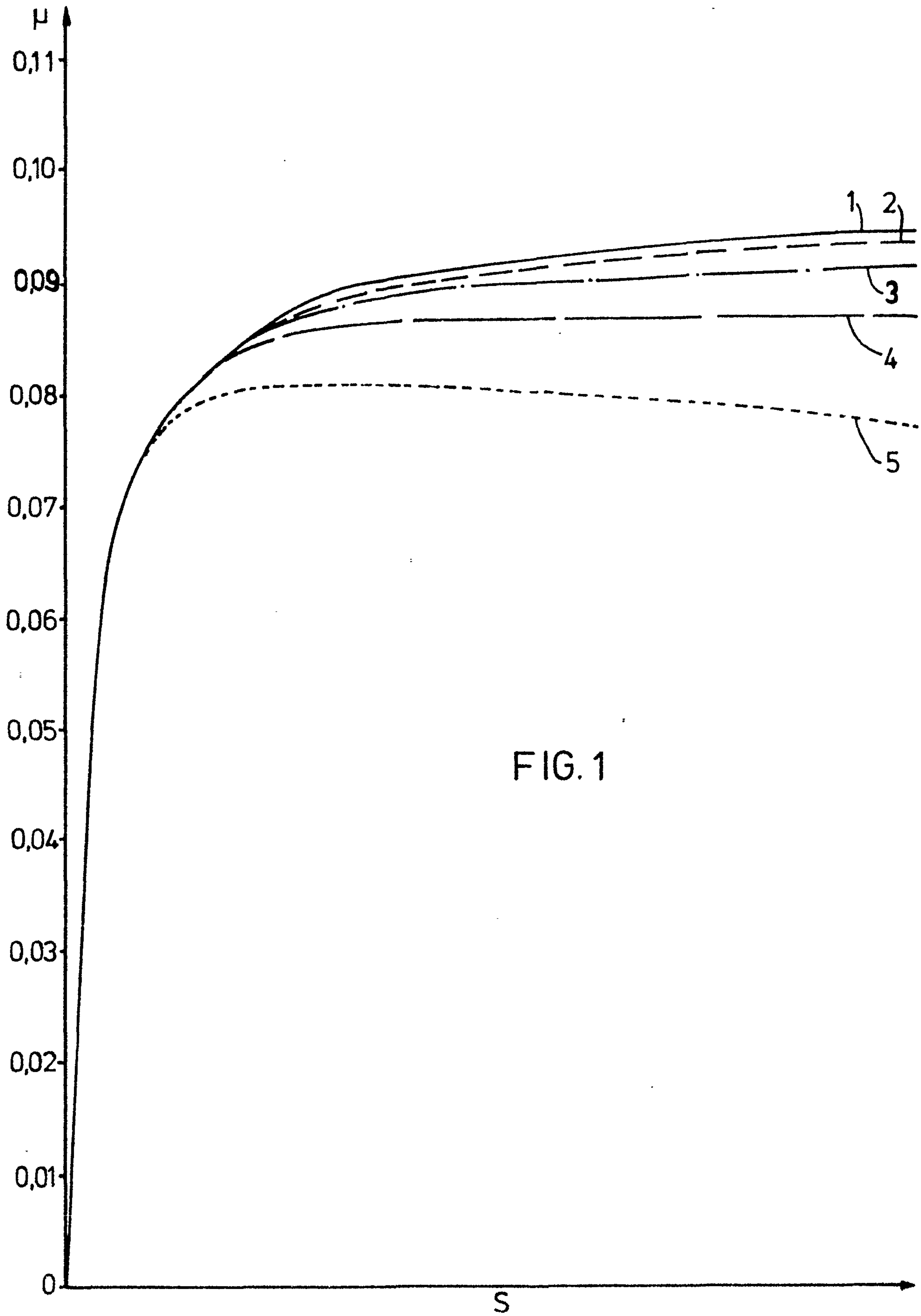


FIG. 1

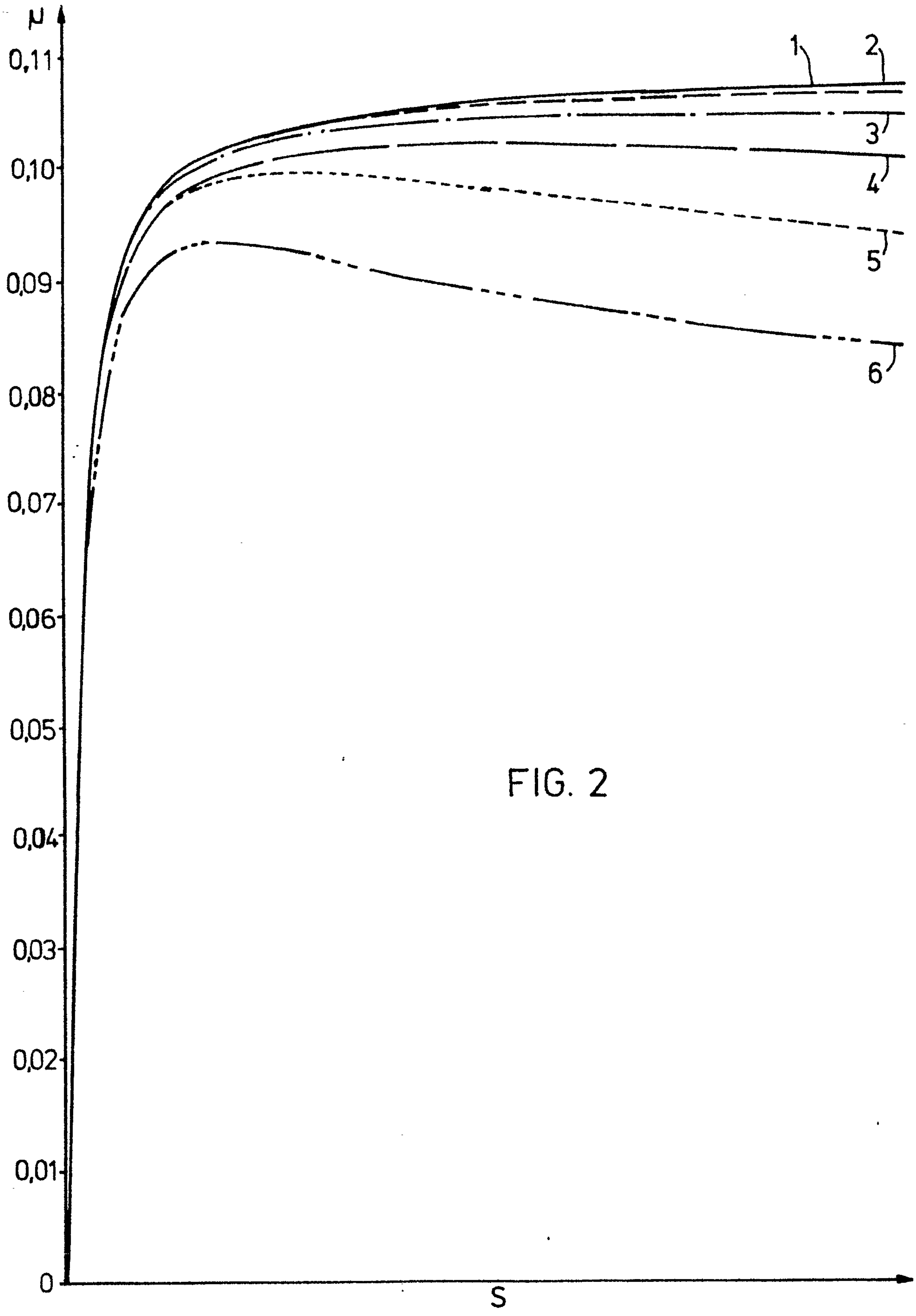


FIG. 2

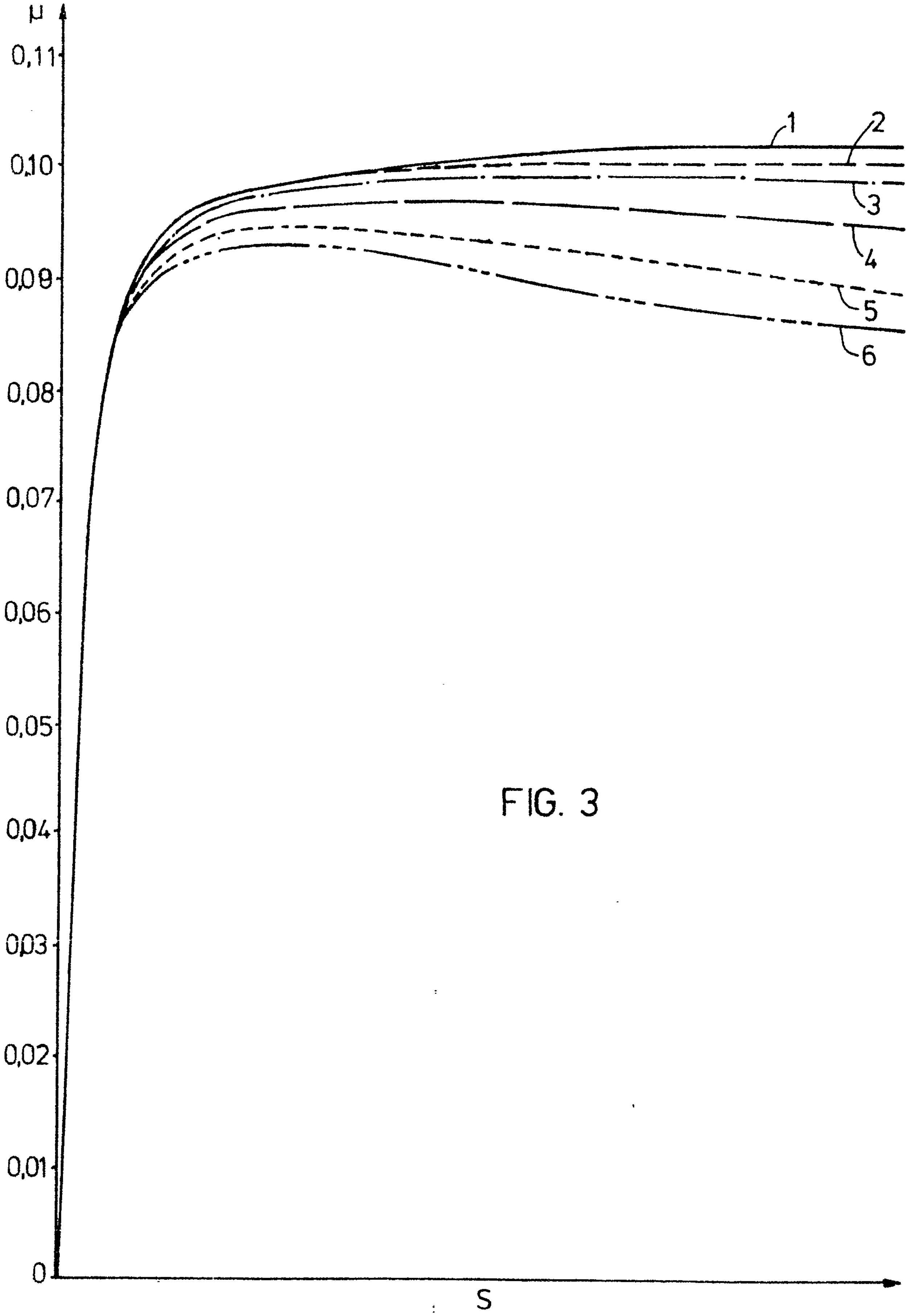


FIG. 3

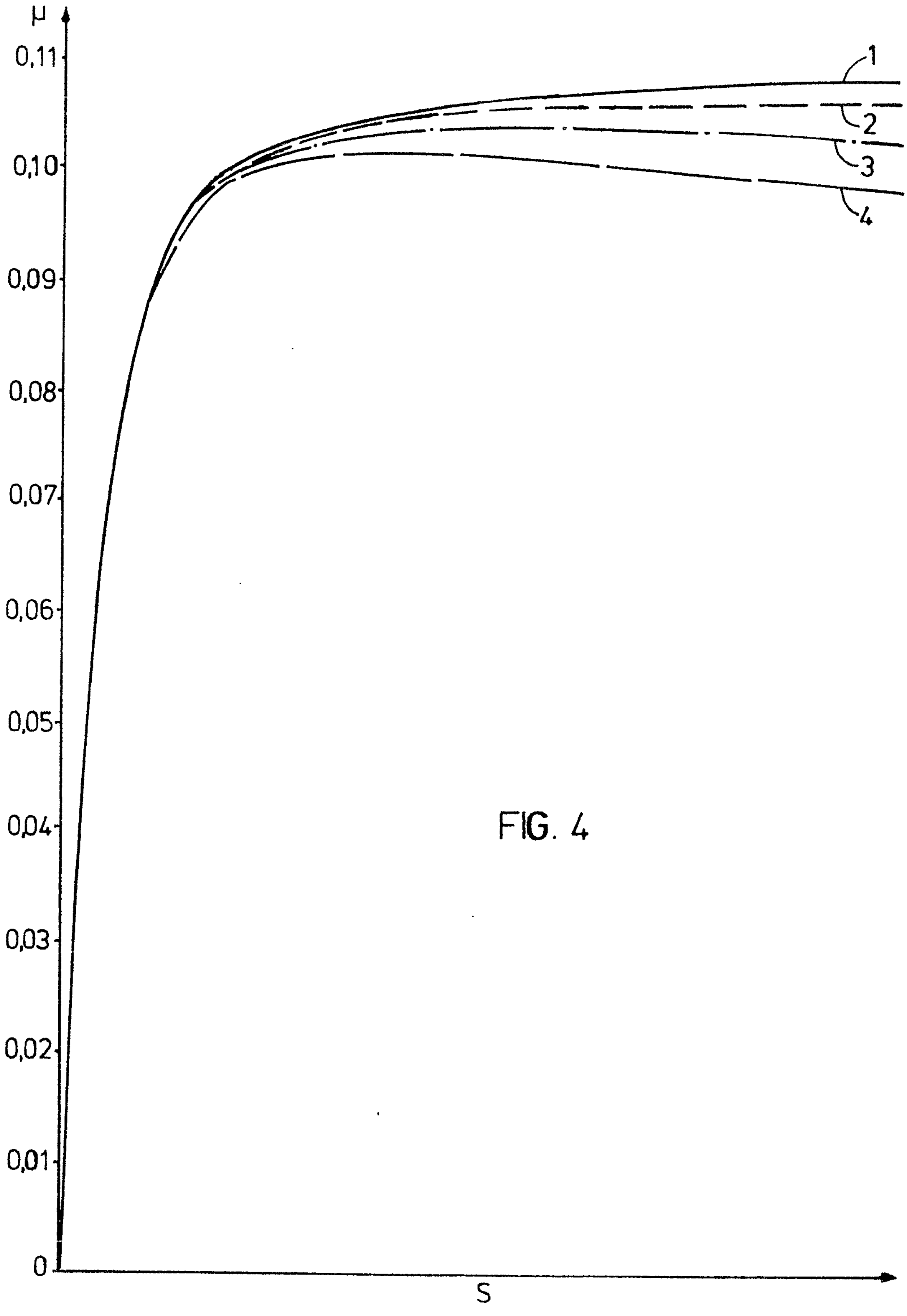


FIG. 4